# FASCICULE DE BREVET EUROPEEN

(11) **EP 3 794 117 B1**
(45) Date de publication et mention de la délivrance du brevet: **15.04.2026**
(21) Numéro de dépôt: 19728105.8
(22) Date de dépôt: 14.05.2019
(51) Int. Cl.: C12N 13/00

(54) **PROCÉDÉ D'OBTENTION DE COMPOSÉS LIPOSOLUBLES ET HYDROSOLUBLES À PARTIR DE MICROALGUES PAR MODULARITÉ DE LA POLARITÉ DES HUILES VÉGÉTALES OU ANIMALES**
VERFAHREN ZUR GEWINNUNG FETTLÖSLICHER UND WASSERLÖSLICHER VERBINDUNGEN AUS MIKROALGEN DURCH MODULATION DER POLARITÄT VON PFLANZLICHEN ODER TIERISCHEN ÖLEN
METHOD FOR OBTAINING FAT-SOLUBLE AND WATER-SOLUBLE COMPOUNDS FROM MICROALGAE BY MODULATING THE POLARITY OF VEGETABLE OR ANIMAL OILS

(30) Priorité: 14.05.2018 FR 1870559
(43) Date de publication de la demande: 24.03.2021
(73) Titulaire: Algama, 75002 Paris (FR); Université d'Avignon et des Pays de Vaucluse, 84029 Avignon Cedex 1 (FR)
(72) Inventeur: CHEMAT, Farid, 84310 MORIERES-LES-AVIGNON (FR); VIAN, Maryline, 30390 ARAMON (FR); VERNES, Léa, 84130 LE PONTET (FR); FELICE, Thomas, 92140 CLAMART (FR)
(74) Mandataire: Touroude, Magali Linda
(86) Numéro de dépôt international: PCT/FR2019/000074
(87) Numéro de publication internationale: WO 2019/220025

(56) Documents cités:
- WO-A1-2010/112760
- WO-A1-94/02161
- FR-A1- 2 694 300
- FR-A1- 2 986 534
- XINSHENG FANG ET AL: "Simultaneous extraction of hydrosoluble phenolic acids and liposoluble tanshinones fromby an optimized microwave-assisted extraction method", SEPARATION AND PURIFICATION TECHNOLOGY, ELSEVIER SCIENCE, AMSTERDAM, NL, vol. 86, 29 October 2011 (2011-10-29), pages 149 - 156, XP028453003, ISSN: 1383-5866, [retrieved on 20111117], DOI: 10.1016/J.SEPPUR.2011.10.039
- CLARA GROSSO ET AL: "Alternative and Efficient Extraction Methods for Marine-Derived Compounds", MARINE DRUGS, vol. 13, no. 5, 21 May 2015 (2015-05-21), pages 3182 - 3230, XP055204822, DOI: 10.3390/md13053182

## Description

La présente invention se rapporte au domaine de la valorisation de la biomasse algale. La présente invention a pour objet un procédé d'obtention de composés liposolubles et hydrosolubles à partir d'une biomasse de microalgues eucaryotes ou procaryotes (cyanobactéries), ainsi que l'huile obtenue par ce procédé et ses applications notamment dans le domaine alimentaire ou comme compléments alimentaires.

Cultivées depuis plus de 30 ans, les microalgues constituent un produit alimentaire à part entière. En nutrition humaine et animale, deux approches de valorisation existent : la première consiste à viser une consommation de la microalgue entière, la deuxième concerne l'extraction, la transformation et le conditionnement de molécules bioactives issues de celles-ci. En France, seules trois espèces de microalgues sont autorisées pour une consommation humaine sans transformation et détiennent des propriétés nutritionnelles exceptionnelles : la spiruline ou Arthrospira platensis, la microalgue verte Chlorella et la diatomée Odontella aurita. Ces micro-organismes s'annoncent comme des éléments majeurs dans l'enjeu de l'alimentation mondiale, grâce à leur teneur unique en protéines et au faible impact environnemental de leur production : faible consommation en eau et en énergie.

Aujourd'hui, les consommateurs se tournent de plus en plus vers une alimentation plus saine et naturelle. Ils recherchent des produits de qualité pour composer leurs repas du quotidien. Parallèlement à ce mode de consommation, un nombre grandissant de personnes recourt à des médecines dites alternatives faisant notamment intervenir les remèdes de grand-mère afin de se soigner le plus naturellement possible. Cependant, une alimentation équilibrée n'est parfois pas suffisante pour éviter les carences. Ainsi les compléments alimentaires sont apparus et se sont largement démocratisés depuis le décret « plantes » de 2014 permettant l'emploi de nombreuses plantes auparavant réservées à la pharmaceutique. Les consommateurs bénéficient désormais d'un large panel de produits en prévention de problèmes sanitaires diverses tels que la prévention de l'obésité, des problèmes cardiovasculaires, l'amélioration de la concentration, ... Les oméga 3 et 6 font partie des molécules en vogue pour leur intérêt dans la santé et leur large gamme d'applications. Ils sont notamment employés en prévention des maladies cardiovasculaires. On les retrouve dans les poissons gras et les fruits de mer, mais ils sont également présents en quantité non négligeable dans les microalgues. Ces acides gras sont dits essentiels car non synthétisés par l'homme ; il faut donc se les procurer via notre alimentation.

L'organisme des mammifères est capable de synthétiser des acides gras à partir de l'acide oléique (C18:1). Cependant, ce n'est pas le cas de certains acides gras polyinsaturés à 34 atomes de carbone qui ne sont synthétisés que par des bactéries ou des végétaux. Les mammifères ne peuvent donc les obtenir que par leur alimentation, c'est pourquoi ces acides gras sont qualifiés d'essentiels. Les deux principaux acides gras essentiels pour l'homme sont : l'acide linoléique et l'acide α-linolénique. C'est à partir de ces acides gras que sont synthétisés d'autres acides gras à chaines longues indispensables pour le bon fonctionnement de l'organisme. Ainsi, par ajout de double liaison et par allongement de la chaine carbonée, on obtient l'acide arachidonique à partir de l'acide linoléique et l'acide eicosapentaénoïque (EPA) à partir de l'acide α-linolénique. Les acides gras synthétisés via ce métabolisme interviennent dans divers processus tels que la signalisation, les réactions inflammatoires, le développement du cerveau et de la rétine... Ce sont les omega 3 (EPA, DHA).

L'extraction de lipides, et notamment d'acides gras essentiels est fréquemment réalisée dans des solvants organiques issus de la pétrochimie tel que l'hexane (Comparison of solvents for extraction of krill oil from krill meal: Lipid yield, phospholipids content, fatty acids composition and minor components. Xie et al, 2017). Ces solvants organiques ont cependant pour inconvénient majeur d'être impropre à la consommation.

Des industriels emploient des corps gras végétaux comme solvant pour extraire des principes actifs liposolubles de plante. Ils sont à l'origine de nombreux brevets tels que par exemple le brevet FR3002845 B1 qui protège un procédé de préparation d'un produit cosmétique et/ou dermatologique à base d'un extrait de liège, d'au moins une partie de l'arbre fournissant le liège, et d'au moins un corps gras naturel, ou encore le brevet FR2994840 B1 qui protège un extrait huileux obtenu par extraction au moyen d'un véhicule huileux de composés non volatiles contenus dans la propolis. D'autres brevets dans un domaine similaire ont vu le jour comme notamment le brevet FR2694300 B1 portant sur un procédé d'extraction et de fixation de composés aromatiques sur un substrat non aqueux.

Cependant, aucun de ces documents ne décrit un procédé modulable permettant d'extraire à la fois les composés liposolubles et les composés hydrosolubles, en particulier à partir d'une biomasse de microalgues, dont le produit peut être utilisé directement pour la consommation humaine ou animale.

Les Demandeurs ont aujourd'hui mis au point un procédé qui permet l'obtention à l'échelle industrielle à partir d'une biomasse de microalgues de départ d'une huile enrichie en tous les composés liposolubles et hydrosolubles de microalgues, soit non seulement les composés liposolubles, mais également les composés hydrosolubles. Le procédé selon l'invention permet en effet grâce à la modulation des paramètres d'extraction et de la polarité de l'huile d'obtenir en outre des composés apolaires, les composés moins apolaires tels que des protéines, tout en contrôlant leur présence. Par ailleurs, le solvant employé étant une huile naturelle, sans trace résiduelle de solvant organique, l'extrait est pur et propice à la consommation alimentaire tel quel et aucune étape d'évaporation n'est nécessaire.

Ainsi un premier objet de la présente invention concerne un procédé d'obtention de composés liposolubles et hydrosolubles à partir d'une biomasse de microalgues eucaryotes ou procaryotes (cyanobactéries), caractérisé en ce que ledit procédé comprend
1. une étape de mélange de ladite biomasse avec de l'huile, de préférence végétale, ladite huile comprenant entre 0.25% et 10% en masse d'au moins un adjuvant amphiphile, choisi parmi un monoglycéride, un diglycéride, ou un phospholipide, permettant de moduler la polarité de ladite huile, et
3. une étape d'extraction desdits composés liposolubles et hydrosolubles par un traitement d'ultrasons avec application d'une puissance ultrasonore (Pus) comprise entre 1 et 1000 W/L appliquée à ladite biomasse en mélange avec ladite huile obtenue à l'étape 1) à une température (T) comprise entre 15 et 70°C pendant une durée comprise entre 30 secondes et 1h, et/ou
4. une étape d'extraction desdits composés liposolubles et hydrosolubles par traitement aux micro-ondes électromagnétiques avec application d'une puissance (W) comprise entre 1 et 1000 W/L appliquée à ladite biomasse en mélange avec ladite huile obtenue à l'étape 1) à une température (T) comprise entre 15 et 70°C pendant une durée comprise entre 30 secondes et 1h.

De manière préférée selon l'invention, le procédé selon l'invention comprend en outre une étape 2. de macération de ladite biomasse avec ladite huile permettant d'homogénéiser le mélange, ladite étape 2. étant réalisée entre l'étape 1. et l'étape 3 ou entre l'étape 1. et l'étape 4 lorsque ledit procédé ne comprend pas d'étape 3.

A titre de précision, le procédé selon l'invention peut comprendre :
- L'étape de mélange 1) suivie par une étape de traitement d'ultrasons 3)
- L'étape de mélange 1) suivie par une étape de traitement aux micro-ondes électromagnétiques 4)
- L'étape de mélange 1) suivie par une étape de macération 2) et une étape de traitement d'ultrasons 3)
- L'étape de mélange 1) suivie par une étape de macération 2) et une étape de traitement aux micro-ondes électromagnétiques 4)
- L'étape de mélange 1) suivie par une étape de traitement d'ultrasons 3) et une étape de traitement aux micro-ondes électromagnétiques 4)
- L'étape de mélange 1) suivie par une étape de macération 2) une étape de traitement d'ultrasons 3), et une étape de traitement aux micro-ondes électromagnétiques 4)

De manière plus préférée selon l'invention, le procédé selon l'invention comprend :
- L'étape de mélange 1) suivie par une étape de macération 2) et une étape de traitement d'ultrasons 3), ou
- L'étape de mélange 1) suivie par une étape de macération 2) et une étape de traitement aux micro-ondes électromagnétiques 4), ou
- L'étape de mélange 1) suivie par une étape de traitement d'ultrasons 3) et une étape de traitement aux micro-ondes électromagnétiques 4), ou
- L'étape de mélange 1) suivie par une étape de macération 2) une étape de traitement d'ultrasons 3), et une étape de traitement aux micro-ondes électromagnétiques 4).

Par composés liposolubles, on entend désigner selon la présente invention des composés solubles dans les corps gras et contenus dans les microalgues et/ou les cyanobactéries, de préférence choisi parmi les acides gras saturés à chaine courte tels que l'acide butyrique, l'acide caproïque, ou l'acide caprylique, les acides gras saturés à chaine longue tel que l'acide Caprique, l'acide Undécanoïque, l'acide Laurique, l'acide Tridécanoïque, l'acide Myristyique, l'acide Pentadécanoïque, l'acide Palmitique, l'acide Margarique ou l'acide Stéarique, les acides gras saturés à chaine très longue tels que l'acide Arachidique, l'acide Docosanoïque, l'acide Tricosanoïque, ou l'acide Tétracosanoïque, les acides gras monoinsaturés tels que l'acide Undecenoïque, l'acide Lauroléique, l'acide Pentadécénoïque, l'acide Palmitoléique, l'acide Heptadécénoïque, l'acide Oléique, l'acide Eicosique, l'acide Docosaénoïque, ou l'acide Tétracosénoïque, les acides gras polyinsaturés tel que l'acide Hexadécadiénoïque, l'acide Linoléique (précurseur des oméga 3 et 6), l'acide Alpha-linolénique (ALA, précurseur des oméga 3 et 6), l'acide Gamma-linolénique (GLA), l'acide Eicosadiénoïque, l'acide Dihomolinolénique, l'acide Eicosapentaenoïque (EPA), les vitamines liposolubles telles que **l'** α-tocophérol (vitamine E), les pigments liposolubles tels que la chlorophylle, ou les caroténoides tels que le β-carotène (précurseur vitamine A), la lutéine, l'Asthaxanthine, la Zeaxanthine, la Cryptoxanthine (précurseur vitamine A), le Lycopène, les stérols, les alcaloïdes, les composés phénoliques tel que les flavonoïdes, ou les terpènes tel que le phytol (précurseur de la vitamine E).

Par composés hydrosolubles, on entend désigner selon la présente invention des composés solubles dans l'eau et contenus dans les microalgues et/ou les cyanobactéries, de préférence choisi parmi les protéines et les enzymes, les vitamines des groupes B et C, les minéraux tel que le zinc, le calcium, le potassium, le magnésium et le fer, les pigments tels que la phycocyanine, l'allophycocyanine, ou la phycoerythrine, et les acides aminés essentiels.

Par microalgues, on entend désigner selon la présente invention les microalgues eucaryotes, qui sont caractérisées par une paroi cellulaire et un noyau, comprenant les chlorophytes, les chrysophytes et les pyrrophytes, lesdites microalgues eucaryotes étant communément dénommées « microalgues », et les microalgues procaryotes, qui ne possèdent pas de noyau et de paroi cellulaire, comprenant les cyanophytes, ci-après dénommées spécifiquement « cyanobactéries ».

Par huile, on entend désigner selon l'invention une substance grasse, liquide à température ordinaire et insoluble dans l'eau, d'origine végétale, animale ou minérale, et compatible pour un usage alimentaire. Cette huile étant constituée essentiellement de triglycéride, celle-ci est complètement apolaire et ne permet initialement d'extraire que les composés de même nature qu'elle, c'est-à-dire les composés très apolaires.

De manière préférée, l'huile utilisée selon l'invention est une huile végétale.

De manière plus préférée, l'huile utilisée selon l'invention est choisie parmi l'huile de noix de coco, l'huile de maïs, l'huile de coton, l'huile d'olive, l'huile de palme, l'huile d'arachide, l'huile de colza, l'huile de canola, l'huile de carthame, l'huile de sésame, l'huile de soja, l'huile de tournesol, l'huile d'amande, l'huile de noix de hêtre, l'huile de noix du brésil, l'huile de noix de cajou, l'huile de noisette, l'huile de macadamia, l'huile de noix de mongongo, l'huile de noix de pécan, l'huile de noix de pin, l'huile de pistache, l'huile de noix, l'huile de graines de citrouille, l'huile de pépins de pamplemousse, l'huile de citron, l'huile d'orange, huile de courge amère, l'huile de courge de buffle, l'huile de graines de courge musquée, l'huile de graines d'egusi, l'huile de graines de citrouille, l'huile de graines de pastèque, l'huile d'açai, l'huile de graine noire, l'huile de graine de cassis, l'huile de graines de bourrache, l'huile d'onagre, l'huile de lin, l'huile d'amarante, l'huile d'abricot, l'huile de pépins de pomme, l'huile d'argan, l'huile d'avocat, l'huile de babassu, l'l'huile de ben, l'huile de noix de suif de bornéo, l'huile de châtaigne du cap, l'huile de gousse de caroube (huile d'algaroba), l'huile de beurre de cacao, l'huile de Lampourde glouteron, l'huile de cohune, l'huile de coriandre, l'huile de cameline, l'huile de pépins de raisin, l'huile de chanvre, l'huile de graines de kapok, l'huile de graines de kénaf, l'huile de lallemantia, l'huile de mafura, l'huile de marula, l'huile de graines de limnanthe, l'huile de moutarde, l'huile de graines de niger, l'huile de graines de coquelicot, l'huile de noix de muscade, l'huile de graines de gombo, l'huile de graines de papaye, l'huile de graines de périlla, l'huile de graines de kaki, l'huile de pequi, l'huile de noix de pili, l'huile de graine de grenade, l'huile de pavot, l'huile de pracaxi, l'huile de pracaxi vierge, l'huile de noyau de pruneau, l'huile de quinoa, l'huile de ramtil, l'huile de son de riz, l'huile royale, l'huile de noix de karité, l'huile de sacha inchi, l'huile de sapote, l'huile de seje, l'huile de beurre de karité, l'huile de taramira, l'huile de graines de thé, l'huile de chardon, l'huile de tigernuts, l'huile de graines de tabac, l'huile de graines de tomates, et l'huile de germes de blé.

De manière encore plus préférée, l'huile utilisée selon l'invention est l'huile de tournesol.

Par adjuvant amphiphile, on entend un composé possédant une partie liposoluble (longue chaine carbonée) et une partie hydrosoluble (ionique ou non). Ces molécules ont la particularité de se concentrer et de s'agréger aux interfaces entre l'eau et d'autres substances peu solubles dans l'eau tels que les corps gras notamment grâce à leurs groupements libres hydroxyde. Les tensioactifs sont des exemples d'adjuvants amphiphiles, et sont classés selon l'indice HLB (Hydrophilic/Lipophilic Balance), l'indice 1 correspondant à l'acide oléique et l'indice 20 à l'oléate de potassium. Cette échelle permet d'apprécier le caractère plutôt liposoluble ou au contraire plutôt hydrophile d'un amphiphile et de savoir pour quelle application il est le mieux adapté. Un tensio-actif ayant un HLB <9 sera de nature liposoluble (en faveur des émulsions eau dans huile) alors qu'un tensio-actif avec un HLB > 11 sera de nature hydrophile (en faveur des émulsions huile dans eau). L'utilisation d'adjuvants amphiphiles ayant des HLB différents permet de moduler la polarité de l'huile utilisée dans le procédé selon l'invention et d'extraire des composés moins apolaires.

De manière préférée, le ou les adjuvants amphiphiles selon l'invention sont choisies parmi les monoglycerides tels que la Monostéarine (E471), le Monooléate de sorbitane (Span 80, E494), le Monolaurate de sorbitane (Tween 20, E 432), le Monopalmitate de sorbitane polyoxyéthylène (Tween 40, E434), le Monooléate de sorbitane polyoxyéthylène (Tween 80 ou polysorbate 80, E433), le Monooléate de glycéryle (type 40, E491), les mono et diglycérides d'acides gras tels que le Palmitostéarate de glycéryle (E471), et les phosphatidylcholines tels que la Lécithine de soja (E322)

Par modulation de la polarité de l'huile, on entend modifier la répartition des charges négatives et positives de l'huile, ce qui permet en réduisant le caractère apolaire de l'huile de favoriser l'extraction de composés polaires (hydrosolubles), ou en l'augmentant de favoriser l'extraction de composés apolaires (liposolubles).

De manière préférée selon l'invention, la biomasse de microalgues eucaryotes ou procaryotes (cyanobactéries) est sous forme sèche, humide ou pré-extraite.

Par biomasse sous forme sèche, on entend désigner selon l'invention de la biomasse ayant subi un séchage ou une lyophilisation. Traditionnellement la biomasse sous forme sèche a un taux d'humidité d'environ 10% d'eau en poids, mais le taux d'humidité peut être plus important si le séchage a été moins long. L'étape de séchage peut être réalisée par toute méthode connue de l'enseignement général de l'Homme du métier, tel que le séchage thermique, consistant par exemple à sécher la biomasse par de la chaleur générée par combustion de gaz naturel (conduction, convection...), soit encore le séchage solaire consistant à utiliser les rayons du soleil pour chauffer et sécher la biomasse par exemple dans des bassins extérieurs. Des exemples de conditions de réalisations de ces étapes de séchages peuvent être trouvés dans le document « Les Différentes techniques de récolte de micro-algues : aspects techniques, économiques et environnementaux. » de Florian Delrue édité dans le cadre du séminaire Algo'Réso le 22 octobre 2013 et disponible sur https://www.mio.univ-amu.fr/IMG/pdf/20131022_02_presa_CEA.pdf

Par biomasse sous forme humide, on entend désigner selon l'invention une biomasse n'ayant pas subi d'étape de séchage, et contenant donc un taux d'humidité important, de l'ordre de 75% d'eau en poids.

Par biomasse sous forme pré-extraite, on entend désigner selon l'invention une biomasse ayant subi une extraction préalable, en particulier de composés hydrosolubles, par exemple par une étape d'extraction mécanique. Cette biomasse pré-extraite peut prendre la forme d'un culot lyophilisé.

De manière préférée selon l'invention, dans le cas où le procédé comprend une étape 3) de traitement ultrasons, la puissance ultrasonore et la température sont modulées pour faire varier la nature et la quantité des composés extraits de ladite biomasse.

Par traitement d'ultrasons avec application d'une puissance ultrasonore (Pus) on entend désigner selon la présente invention un traitement physique mettant en œuvre un réacteur à ultrasons. De manière préférée selon l'invention, la puissance ultrasonore est comprise entre 1 et 1000 W/L appliquée aux microalgues ou aux cyanobactéries en mélange avec l'huile et ladite au moins un adjuvant amphiphile, de manière préférée entre 30 et 100 W/L, de manière encore préférée entre 50 et 80 W/L. De manière préférée, l'énergie déployée par le réacteur à ultrasons varie entre 500 et 5000 J, le débit d'alimentation du réacteur à ultrasons varie de 1 L/heure à 1000L/heure, de préférence 1L/heure, et le traitement aux ultrasons est réalisé pendant une durée variant de 30 secondes à 1 heure.

De manière préférée selon l'invention, dans le cas où le procédé comprend une étape 4) de traitement aux micro-ondes électromagnétiques, la puissance, le nombre d'exposition et la température sont modulées pour faire varier la nature et la quantité des composés extraits de ladite biomasse.

De manière préférée selon l'invention, la modulation de la puissance des micro-ondes électromagnétiques permet d'augmenter ou de réduire la quantité de composés liposolubles et hydrosolubles extraits de ladite biomasse, chacun desdits composés ayant une puissance optimale où un maximum de celui-ci est extrait, par exemple 850W pour la chlorophylle a et 1000 W pour les caroténoïdes. Utiliser d'autres puissances pour ces composés permettra ainsi d'en réduire la quantité extraite.

Par traitement aux micro-ondes électromagnétiques, on entend désigner selon la présente invention un traitement de la biomasse à des ondes électromagnétiques imposées par un générateur de micro-ondes.

Par nombre d'exposition, on entend désigner le nombre de répétitions du traitement par micro-ondes.

De manière préférée selon l'invention, le traitement prend la forme d'au moins une exposition de 30 secondes à 30 minutes. De manière encore plus préférée selon l'invention, le traitement prend la forme de plusieurs expositions chacune durant de 30 secondes à 30 minutes.

De manière préférée selon l'invention, la forme de la biomasse d'origine, la nature et quantité de l'huile et dudit au moins un adjuvant amphiphile, et l'étape 3) de traitement ultrasons et/ou 4) de traitement aux micro-ondes électromagnétiques sont modulées pour faire varier la nature et la quantité des composés liposolubles et hydrosolubles extraits desdites microalgues eucaryotes ou aux cyanobactéries.

La modulation de la forme de la biomasse d'origine permet d'augmenter ou de réduire la quantité de composés liposolubles et hydrosolubles extraits de ladite biomasse. L'utilisation d'une biomasse pré-extraite permet d'extraire plus de composés qu'une biomasse sèche, l'utilisation de cette dernière permettant d'extraite davantage de composés qu'avec une biomasse humide.

La modulation par la nature et la quantité d'adjuvant amphiphile affecte les rendements d'extraction des composés liposolubles et hydrosolubles quelle que soit la technique utilisée. L'ajout par exemple de Tween 80 ou de Span 80 permet par traitement à des ultrasons ou par traitement aux micro-ondes d'augmenter la quantité de composés extraits. Par traitement par macération a également été caractérisé que l'ajout de Span 80 permet d'augmenter le rendement d'extraction de caroténoïdes et de chlorophylle a, alors que l'ajout de Tween 80 ne permet d'augmenter que le rendement d'extraction de chlorophylle a, le rendement d'extraction de caroténoïdes diminuant.

La modulation de l'étape 3) de traitement ultrasons permet d'augmenter ou de réduire la quantité de composés liposolubles et hydrosolubles extraits de ladite biomasse, chacun desdits composés ayant une puissance ultrasonore optimale où un maximum de celui-ci est extrait.

La modulation de l'étape 4) de traitement aux micro-ondes électromagnétiques permet d'augmenter ou de réduire la quantité de composés liposolubles et hydrosolubles extraits de ladite biomasse, chacun desdits composés ayant une puissance optimale où un maximum de celui-ci est extrait, par exemple 850W pour la chlorophylle a et 1000 W pour les caroténoïdes. Utiliser d'autres puissances pour ces composés que cette puissance optimale conduira ainsi à en réduire la quantité extraite.

De manière préférée selon l'invention, la durée de l'étape 3) et/ou de l'étape 4) est chacune comprise entre 30 secondes et 1h.

De manière encore plus préférée selon l'invention, la durée de l'étape 3) et de l'étape 4) est chacune comprise 30 secondes à 30 minutes.

De manière préférée selon l'invention, les composés liposolubles sont choisis parmi les acides gras saturés à chaine courte tels que l'acide butyrique, l'acide caproïque, ou l'acide caprylique, les acides gras saturés à chaine longue tel que l'acide Caprique, l'acide Undécanoïque, l'acide Laurique, l'acide Tridécanoïque, l'acide Myristyique, l'acide Pentadécanoïque, l'acide Palmitique, l'acide Margarique ou l'acide Stéarique, les acides gras saturés à chaine très longue tels que l'acide Arachidique, l'acide Docosanoïque, l'acide Tricosanoïque, ou l'acide Tétracosanoïque, les acides gras monoinsaturés tels que l'acide Undecenoïque, l'acide Lauroléique, l'acide Pentadécénoïque, l'acide Palmitoléique, l'acide Heptadécénoïque, l'acide Oléique, l'acide Eicosique, l'acide Docosaénoïque, ou l'acide Tétracosénoïque, les acides gras polyinsaturés tel que l'acide Hexadécadiénoïque, l'acide Linoléique (précurseur des oméga 3 et 6), l'acide Alpha-linolénique (ALA, précurseur des oméga 3 et 6), l'acide Gamma-linolénique (GLA), l'acide Eicosadiénoïque, l'acide Dihomolinolénique, l'acide Eicosapentaenoïque (EPA), les vitamines liposolubles telles que l' α-tocophérol (vitamine E), ou les pigments liposolubles tels que la chlorophylle, ou les caroténoides tels que le β-carotène (précurseur vitamine A), la lutéine, l'Asthaxanthine, la Zeaxanthine, la Cryptoxanthine (précurseur vitamine A), ou le Lycopène.

De manière préférée selon l'invention, les composés hydrosolubles sont choisis parmi les protéines et les enzymes, les vitamines des groupes B et C, les minéraux tel que le zinc, le calcium, le potassium, le magnésium et le fer, les pigments tels que la phycocyanine, l'allophycocyanine, ou la phycoerythrine, les acides aminés essentiels, les polysaccharides et les fibres.

De manière préférée selon l'invention, les microalgues eucaryotes sont choisies parmi Chlorella, Nannocloropsis, Dunaliella et Euglena, et les cyanobactéries sont choisies parmi la spiruline (Arthrospira platensis ou Spirulina maxima) et l'AFA (Aphanizomenon Floes-aquae).

De manière préférée selon l'invention, le procédé comprend une étape supplémentaire 5) de séparation solide-liquide, de préférence par centrifugation, de l'extrait obtenu après l'étape 2), 3) ou 4).

De manière préférée selon l'invention, le procédé comprend une étape supplémentaire 6) de filtration de la partie liquide obtenue par séparation de l'extrait obtenu après l'étape 5).

De manière préférée selon l'invention, le rapport microalgues ou cyanobactéries/huile est compris entre 1/5^{ème} et 1/50^{ème} du volume, de préférence 1/20^{ème} du volume.

Selon un deuxième aspect, l'invention concerne une huile enrichie en composés liposolubles et hydrosolubles de microalgues eucaryotes ou procaryotes (cyanobactéries) telle qu'obtenue par le procédé selon l'invention.

Par huile enrichie en composés liposolubles et hydrosolubles de microalgues eucaryotes ou procaryotes (cyanobactéries), on entend une huile comprenant des composés hydrosolubles et liposolubles non-présents initialement dans l'huile, et/ou une augmentation de la concentration de composés hydrosolubles et liposolubles présents initialement dans l'huile en quantité extrêmement faible (mesure inférieure au microgramme).

De manière préférée, l'invention concerne une huile enrichie en composés hydrosolubles et liposolubles non-présents initialement dans l'huile.

Plus particulièrement, ledit procédé permet d'extraire des microalgues et cyanobactéries les composés liposolubles et les composés hydrosolubles. Préférentiellement, l'extrait produit possèdera des qualités organoleptiques ainsi qu'une composition nutritionnelle optimale pour la consommation humaine.

Selon un troisième aspect, l'invention concerne l'utilisation de selon l'invention dans des compositions chimiques, alimentaires, cosmétiques ou pharmaceutiques, de préférence alimentaire.

Compte tenu des avantages évoqués ci-dessus présentés par ces filtrats/extraits, leurs applications dans le domaine alimentaire, médical ou cosmétique, en particulier comme compléments alimentaires, s'avèrent particulièrement intéressantes. L'invention vise également en tant que nouveaux produits les rétentats obtenus à l'issue de l'étape de filtration. Ces rétentats sont également utilisables dans le domaine alimentaire, médical ou cosmétique ou comme compléments alimentaires.

### Description des Figures

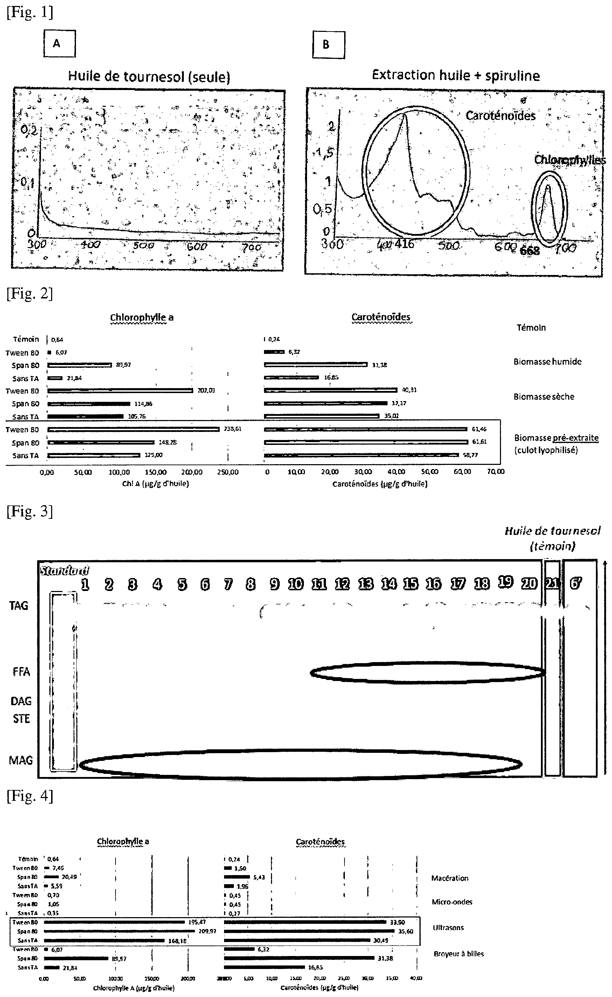

Figure 1 : Spectres d'absorption de l'huile seule (A) et de l'huile enrichie en composés de spiruline (B) après que la biomasse de Arthrospira platensis ait été mélangée au solvant d'extraction (huile végétale de tournesol à 1 % de Tween 80) selon le ratio 1/20 (0.5 g de spiruline dans 10 g d'huile) et que le mélange ait été introduit dans un broyeur à billes avec des billes de céramique et soumis à des rotations d'une vitesse de 4000 tour/minute durant 1h à température ambiante, puis centrifugés 10 min à 9000 rpm.
Figure 2 : Analyse quantitative réalisée par mesures spectrophotométriques afin d'évaluer la teneur en chlorophylle a et en caroténoïdes en fonction de la nature de la biomasse (humide, sèche ou pré-extraite) et de la nature de l'adjuvant amphiphile (aucun, du Tween 80, ou du Span 80) après avoir introduit le mélange 1h dans un broyeur à billes à 4000 rpm à température ambiante. La première ligne présente une valeur témoin. Les 3 lignes suivantes présentent les données après utilisation de biomasse humide. Les 3 lignes suivantes présentent les données après utilisation de biomasse sèche. Les 3 lignes suivantes présentent les données après utilisation de biomasse pré-extraite, en l'occurrence un culot lyophilisé.
Figure 3 : Analyse qualitative des lipides neutres réalisée par chromatographie haute performance sur couche mince (HPTLC). 20 µL d'échantillon à 1 mg/mL ont été déposés dans du choloroforme. La présence de monoglycerides (MAG), d'acides gras libres (FFA), de triglycérides (TAG), de diglycerides (DAG) et de stérols (STE) est étudiée.
Figure 4 : Analyse quantitative réalisée par mesures spectrophotométriques afin d'évaluer la teneur en chlorophylle a et en caroténoïdes en fonction de la technique d'extraction (broyeur à billes, macération, micro-ondes ou ultrasons) et de la nature de l'adjuvant amphiphile (aucun, du Tween 80, ou du Span 80) en utilisant une biomasse humide (80% d'humidité). La première ligne présente une valeur témoin. Les 3 lignes suivantes présentent les données après macération pendant 24 heures. Les 3 lignes suivantes présentent les données après traitement aux micro-ondes électromagnétiques pendant 4 fois une minute. Les 3 lignes suivantes présentent les données après traitement par ultrasons avec application d'une puissance ultrasonore pendant 30 minutes. Les 3 dernières lignes présentent les données après traitement par un broyeur à billes pendant 1 heure.

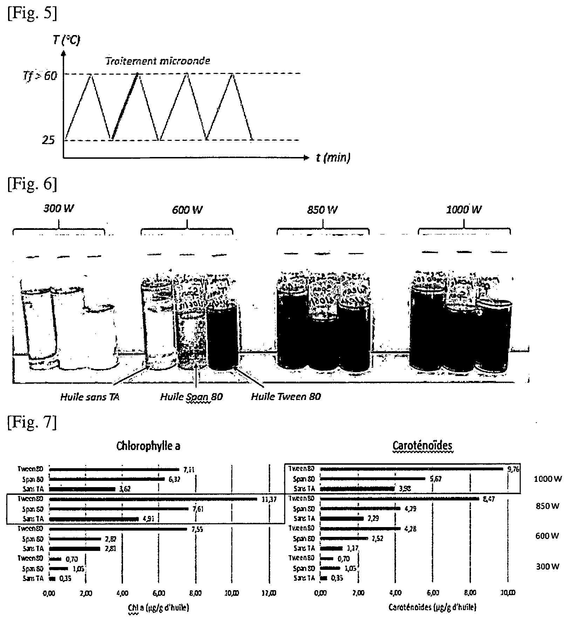

Figure 5 : Schématisation des cycles d'application des micro-ondes. Les courbes montantes représentent les moments de traitement par micro-ondes.
Figure 6 : Photos caractérisant les extractions réalisées par traitement par micro-ondes à des puissances de 300, 600, 850 et 1000 W et selon la nature de l'adjuvant amphiphile (aucun, du Tween 80, ou du Span 80).
Figure 7 : Analyse quantitative réalisée par mesures spectrophotométriques afin d'évaluer la teneur en chlorophylle a et en caroténoïdes en fonction de la puissance du traitement par micro-ondes (300, 600, 850 et 1000 W). Les 3 premières lignes présentent les données avec un traitement à 1000 W. Les 3 lignes suivantes présentent les données avec un traitement à 850 W. Les 3 lignes présentent les données avec un traitement à 600 W. Les 3 dernières lignes présentent les données avec un traitement à 300 W.

### Exemples

### Exemples 1 : Etude de la modularité de l'état de la biomasse et de l'ajout d'adjuvant amphiphile par enrichissement de l'huile de tournesol par l'utilisation d'un broyeur à billes :

La biomasse (*Arthrospira platensis)* humide, sèche ou pré-extraite est mélangée au solvant d'extraction (huile végétale de tournesol, huile végétale de tournesol à 1 % de Tween 80 et huile végétaile de tournesol à 1 % de Span 80) selon le ratio 1/20 ; soit 1 g de biomasse pour 20 g de solvant. Dans ce cas, 0.5 g de spiruline sont introduits dans 10 g d'huile. Les mélangessont ensuite introduits dans un broyeur à billes avec des billes de céramique et soumis à des rotations d'une vitesse de 4000 tour/minute durant 1h à température ambiante. Les extraits sont ensuite centrifugés 10 min à 9000 rpm afin d'obtenir une huile limpide.

L'huile collectée après ces extractions présente une couleur verte, différente de la couleur initiale de l'huile de tournesol de départ (jaune clair). La comparaison des spectres d'absorption de l'huile seule (Figure 1A) et de l'huile végétale de tournesol à 1 % de Tween 80 enrichie en composés de spiruline (Figure 1B) révèle la présence de pics caractéristiques de pigments végétaux : les caroténoïdes (λₘₐₓ= 416 nm) et la chlorophylle (λₘₐₓ= 668 nm). D'après ces premières observations qualitatives, l'huile de tournesol aurait donc été enrichie en pigments de spiruline par le procédé selon l'invention.

Une analyse quantitative a ensuite été réalisée via des mesures spectrophotométriques afin d'évaluer la teneur en chacun de ses pigments en fonction de la nature de la biomasse et de l'adjuvant amphiphile. Les résultats obtenus (Figure 2) mettent en évidence le fait que la nature de la biomasse module la teneur en pigments. En effet, les teneurs maximales en pigments sont atteintes avec une biomasse pré-extraite (culot lyophilisé : ayant déjà subi une extraction). La modulation de la polarité par l'ajout d'adjuvant amphiphile dans l'huile affecte également les rendements en pigments. Ainsi avec le tensio-actif Tween 80, l'extrait contient 238.61 µg de chlorophylle a par g d'huile et 61.46 µg de caroténoïdes par g d'huile.

Par ailleurs, si l'on se focalise sur les classes de lipides, on constate également l'enrichissement de l'huile végétale en lipides neutre de spiruline après extraction. Une analyse qualitative des lipides neutres a été mise en œuvre (Figure 3) via une chromatographie haute performance sur couche mince (HPTLC) où on a procédé à un dépôt de 20 µL d'échantillon à 1 mg/mL dans le chloroforme. Les résultats obtenus montrent la présence de monoglycérides (MAG) dans les extraits 1 à 19 et non dans le témoin, et la présence d'acides gras libres (FFA) dans les extraits 12 à 19 et non dans le témoin. Ces résultats mettent en avant la présence de lipides neutres tels que des acides gras libres (FFA) et des monoglycérides (MAG) dans l'huile végétale après extraction. Il y a donc eu un enrichissement de l'huile végétale qui ne contenait pas ces composés à l'état initial.

### Exemple 2 : Etude de la modularité de la technique d'extraction - enrichissement de l'huile de tournesol par différentes méthodes d'extraction

Pour une biomasse donnée (ici biomasse humide (80% d'humidité) d'*Arthrospira platensis*), différentes méthodes d'extraction ont été employées afin d'enrichir l'huile de tournesol :
- Broyeur à billes : 4000 rpm, pendant 1h, à l'aide de 20 g de billes de céramique
- Ultrasons : 25 kHz, 150 W, pendant 30 min
- Micro-ondes : essai avec différentes puissances allant de 300 à 1000 W, par des traitements par cycles d'1 minute
- Macération.

Les mesures spectrophotométriques réalisées (Figure 4) montrent que les rendements en chlorophylle a et en caroténoïdes sont modulés par la technique d'extraction choisie. En effet, les rendements maximaux sont atteints avec une extraction par ultrasons : 209.92 µg de chlorophylle a par g d'huile et 35.60 µg de caroténoïdes par g d'huile (avec de l'huile à 1 % de span 80). Dans ce cas, l'ajout de span 80 dans l'huile module la polarité en faveur de l'extraction des pigments chlorophylle et caroténoïdes.

### Exemple 3 : Etude de la modularité des paramètres d'extraction - enrichissement de l'huile de tournesol par micro-ondes

Afin de mettre en avant la modularité de la technique d'extraction, différents paramètres ont été testés. La biomasse choisie pour ces extractions est une pâte de spiruline à 79.87% d'humidité. Le mélange biomasse/huile est réalisé au ratio sec 1/20^{ème}. Plusieurs cycles d'expositions aux micro-ondes d'une durée d'1 minute ont été appliqués au mélange solvant/biomasse, entrecoupés d'un refroidissement dans un bain de glace (illustré dans la Figure 5).

Des extractions ont été réalisées par traitement par micro-ondes à des puissances de 300, 600, 850 et 1000 W. La figure 6 montre que l'huile devient de plus en plus foncée lorsque la puissance micro-onde augmente, traduisant que l'extraction de pigment est donc favorisée par une puissance micro-onde élevée. Par ailleurs, la modulation de la polarité joue également un rôle important sur l'extraction de pigments puisqu'on constate une différence de couleur en fonction de l'adjuvant amphiphile utilisée pour une même puissance. Ainsi à 600 W, l'huile comprenant du Tween 80 à 1 % présente une couleur plus intense que les extraits réalisés avec de l'huile seule et avec de l'huile additionnée de Span 80 à 1%.

Les résultats présentés en Figure 7 confirment les observations du paragraphe précédent. Plus la puissance micro-onde est élevée, plus le rendement en pigments est important ; de même, l'addition de Tween 80 dans l'huile permet un rendement en pigment supérieur à celui obtenu avec l'huile seule ou bien avec l'huile additionnée de Span 80 : 11.37 µg de chlorophylle a par g d'huile et 9.76 µg de caroténoïdes par g d'huile avec l'huile additionnée de Tween 80 à 1% à 850 et 1000 W respectivement.

### Conclusions

Les résultats obtenus dans ces trois exemples mettent en évidence que la modularité de la nature de la biomasse joue sur la quantité de composés liposolubles et hydrosolubles extraits. En effet, utiliser une biomasse pré-extraite permettra une meilleure extraction des composés liposolubles et hydrosolubles qu'avec une biomasse sèche, cette dernière permettant cependant d'obtenir une meilleure extraction des composés liposolubles et hydrosolubles qu'avec une extraction à partir de biomasse humide.

Les résultats obtenus dans ces trois exemples mettent également en évidence que la technique d'extraction utilisée module les rendements d'extraction des composés liposolubles et hydrosolubles. En effet, l'utilisation d'ultrasons permet d'obtenir des rendements supérieurs d'extraction de caroténoïdes et de chlorophylle a, en comparaison à la macération, le traitement par micro-ondes ou l'utilisation d'un broyeur à billes.

Les résultats obtenus dans ces trois exemples mettent également en évidence que la modulation de la technique basée sur des micro-ondes influence les rendements d'extraction des composés liposolubles et hydrosolubles . En effet, une quantité maximum de caroténoïdes est extrait à la puissance micro-ondes de 1000 W, alors qu'un maximum de chlorophylle a est extraite à 850 W.

Enfin, les résultats obtenus dans ces trois exemples mettent en évidence que l'ajout d'adjuvant amphiphile affecte les rendements d'extraction des composés liposolubles et hydrosolubles quelle que soit la technique utilisée. L'ajout par exemple de Tween 80 ou de Span 80 permet par traitement à des ultrasons ou par traitement aux micro-ondes d'augmenter la quantité de composés extraits. Par traitement par macération a également été caractérisé que l'ajout de Span 80 permet d'augmenter le rendement d'extraction de caroténoïdes et de chlorophylle a, alors que l'ajout de Tween 80 ne permet d'augmenter que le rendement d'extraction de chlorophyle a, le rendement d'extraction de caroténoïdes diminuant..

## Revendications

1. Procédé d'obtention de composés liposolubles et hydrosolubles à partir d'une biomasse de microalgues eucaryotes ou procaryotes, **caractérisé en ce que** ledit procédé comprend : 1. une étape de mélange de ladite biomasse avec de l'huile, de préférence végétale, ladite huile comprenant entre 0.25% et 10% en masse d'au moins un adjuvant amphiphile choisi parmi un monoglycéride, un diglycéride, ou un phospholipide, permettant de moduler la polarité de ladite huile, et 3. une étape d'extraction desdits composés liposolubles et hydrosolubles par un traitement d'ultrasons avec application d'une puissance ultrasonore (Pus) comprise entre 1 et 1000 W/L appliquée à ladite biomasse en mélange avec ladite huile obtenue à l'étape 1) à une température (T) comprise entre 15 et 70°C pendant une durée comprise entre 30 secondes et 1h, et/ou 4. une étape d'extraction desdits composés liposolubles et hydrosolubles par traitement aux micro-ondes électromagnétiques avec application d'une puissance (W) comprise entre 1 et 1000 W/L appliquée à ladite biomasse en mélange avec ladite huile obtenue à l'étape 1) à une température (T) comprise entre 15 et 70°C pendant une durée comprise entre 30 secondes et 1h.

2. Procédé selon la revendication précédente, **caractérisé en ce que** la biomasse de microalgues eucaryotes ou procaryotes est sous forme sèche, humide ou pré-extraite.

3. Procédé selon l'une quelconque des revendications précédentes, où ledit au moins un adjuvant amphiphile est choisi parmi la Monostéarine, le Monooléate de sorbitane, le Monolaurate de sorbitane, le Monopalmitate de sorbitane polyoxyéthylène, le Monooléate de sorbitane polyoxyéthylène, le Monooléate de glycéryle, le Palmitostéarate de glycéryle, ou la Lécithine de soja.

4. Procédé selon la revendication précédente, où ledit au moins un adjuvant amphiphile est choisi parmi le Monooléate de sorbitane ou le Monooléate de sorbitane polyoxyéthylène.

5. Procédé selon l'une quelconque des revendications précédentes, **caractérisée en ce que** les composés liposolubles sont choisis parmi les acides gras saturés à chaine courte tels que l'acide butyrique, l'acide caproïque, ou l'acide caprylique, les acides gras saturés à chaine longue tel que l'acide Caprique, l'acide Undécanoïque, l'acide Laurique, l'acide Tridécanoïque, l'acide Myristyique, l'acide Pentadécanoïque, l'acide Palmitique, l'acide Margarique ou l'acide Stéarique, les acides gras saturés à chaine très longue tels que l'acide Arachidique, l'acide Docosanoïque, l'acide Tricosanoïque, ou l'acide Tétracosanoïque, les acides gras monoinsaturés tels que l'acide Undecenoïque, l'acide Lauroléique, l'acide Pentadécénoïque, l'acide Palmitoléique, l'acide Heptadécénoïque, l'acide Oléique, l'acide Eicosique, l'acide Docosaénoïque, ou l'acide Tétracosénoïque, les acides gras polyinsaturés tel que l'acide Hexadécadiénoïque, l'acide Linoléique (précurseur des oméga 3 et 6), l'acide Alpha-linolénique (ALA, précurseur des oméga 3 et 6), l'acide Gamma-linolénique (GLA), l'acide Eicosadiénoïque, l'acide Dihomolinolénique, l'acide Eicosapentaenoïque (EPA), les vitamines liposolubles telles que l' α-tocophérol (vitamine E), ou les pigments liposolubles tels que la chlorophylle, ou les caroténoides tels que le β-carotène (précurseur vitamine A), la lutéine, l'Asthaxanthine, la Zeaxanthine, la Cryptoxanthine (précurseur vitamine A), le Lycopène, les stérols, les alcaloïdes, les composés phénoliques tel que les flavonoïdes, ou les terpènes tel que le phytol (précurseur de la vitamine E).

6. Procédé selon l'une quelconque des revendications précédentes, **caractérisé en ce que** les microalgues eucaryotes sont choisies parmi Chlorella, Nannocloropsis, Dunaliella et Euglena, et que les microalgues procaryotes sont choisies parmi la spiruline Arthrospira platensis ou Spirulina maxima et l'Aphanizomenon Floes-aquae.

7. Procédé selon l'une quelconque des revendications précédentes, **caractérisé en ce qu'**il comprend une étape supplémentaire 5) de séparation solide-liquide, de préférence par centrifugation, de l'extrait obtenu après l'étape 2), 3) ou 4).

8. Procédé selon la revendication précédente, **caractérisé en ce qu'**il comprend une étape supplémentaire 6) de filtration de la partie liquide obtenue par séparation de l'extrait obtenu après l'étape 5).

9. Procédé selon l'une quelconque des revendications précédentes, **caractérisé en ce que** le rapport microalgues ou cyanobactéries/huile est compris entre 1/5^{ème} et 1/50^{ème} du volume, de préférence 1/20^{ème} du volume.

10. Utilisation du procédé selon l'une quelconque des revendications précédentes, où dans le cas où le procédé comprend une étape 3) de traitement aux ultrasons, la puissance ultrasonore et la température sont modulées pour faire varier la nature et la quantité des composés extraits de ladite biomasse.

11. Utilisation du procédé selon l'une quelconque des revendications 1 à 9, où dans le cas où le procédé comprend une étape 4) de traitement aux micro-ondes électromagnétiques, la puissance, le nombre d'exposition et la température sont modulées pour faire varier la nature et la quantité des composés extraits de ladite biomasse.

12. Utilisation du procédé selon l'une quelconque des revendications 1 à 9, où la forme de la biomasse d'origine, la nature et quantité de l'huile et la nature et quantité dudit au moins un adjuvant amphiphile, et l'étape 3) de traitement ultrasons et/ou 4) de traitement aux micro-ondes électromagnétiques sont modulés pour faire varier les composés liposolubles et hydrosolubles extraits desdites microalgues eucaryotes ou aux cyanobactéries.

## Patentansprüche

1. Verfahren zum Gewinnen von fettlöslichen und wasserlöslichen Verbindungen aus einer Biomasse aus eukaryotischen oder prokaryotischen Mikroalgen, **dadurch gekennzeichnet, dass** das Verfahren umfasst:
1. einen Schritt des Mischens der besagten Biomasse mit Öl, vorzugsweise pflanzlichem Öl, das Öl umfassend zu zwischen 0,25 Gew.-% und 10 Gew.-% mindestens einen amphiphilen Zusatzstoff, ausgewählt aus einem Monoglycerid, einem Diglycerid oder einem Phospholipid, wobei ein Modulieren der Polarität des Öls ermöglicht wird, und
3. einen Extraktionsschritt der fettlöslichen und wasserlöslichen Verbindungen durch eine Ultraschallbehandlung mit Anwendung einer Ultraschallleistung (Pus), die zwischen 1 und 1000 W/L liegt, die auf die Biomasse in der Mischung mit dem Öl, das in Schritt 1) gewonnen wird, bei einer Temperatur (T), die zwischen 15 und 70 °C liegt, während einer Dauer, die zwischen 30 Sekunden und 1 h liegt, angewendet wird, und/oder
4. einen Extraktionsschritt der fettlöslichen und wasserlöslichen Verbindungen durch Behandlung mit elektromagnetischen Mikrowellen unter Anwendung einer Leistung (W), die zwischen 1 und 1000 W/L liegt, die auf die Biomasse in der Mischung mit dem Öl, das in Schritt 1) gewonnen wird, bei einer Temperatur (T), die zwischen 15 und 70 °C liegt, während einer Dauer, die zwischen 30 Sekunden und 1 h liegt, angewendet wird.

2. Verfahren nach dem vorstehenden Anspruch,
**dadurch gekennzeichnet, dass** die Biomasse aus eukaryotischen oder prokaryotischen Mikroalgen in trockener, feuchter oder vorextrahierter Form vorliegt.

3. Verfahren nach einem der vorstehenden Ansprüche, wobei der mindestens eine amphiphile Zusatzstoff ausgewählt ist aus Monostearin, Sorbitanmonooleat, Sorbitanmonolaurat, Polyoxyethylensorbitanmonopalmitat, Polyoxyethylensorbitanmonooleat, Glycerylmonooleat, Glycerylpalmitostearat oder Sojalecithin.

4. Verfahren nach dem vorstehenden Anspruch, wobei der mindestens eine amphiphile Zusatzstoff ausgewählt ist aus Sorbitanmonooleat oder Polyoxyethylensorbitanmonooleat.

5. Verfahren nach einem der vorstehenden Ansprüche,
**dadurch gekennzeichnet, dass** die fettlöslichen Verbindungen ausgewählt sind aus kurzkettigen gesättigten Fettsäuren wie Buttersäure, Capronsäure oder Caprylsäure, langkettigen gesättigten Fettsäuren wie Caprinsäure, Undecansäure, Laurinsäure, Tridecansäure, Myristinsäure, Pentadecansäure, Palmitinsäure, Margarinsäure oder Stearinsäure, sehr langkettigen gesättigten Fettsäuren wie Arachinsäure, Docosansäure, Tricosansäure oder Tetracosansäure, einfach ungesättigten Fettsäuren wie Undecensäure, Lauroleinsäure, Pentadecensäure, Palmitoleinsäure, Heptadecensäure, Ölsäure, Eicosensäure, Docosaensäure oder Tetracosensäure, mehrfach ungesättigten Fettsäuren wie Hexadecadiensäure, Linolsäure (Vorläufer der Omega-3- und -6-Fettsäuren), Alpha-Linolensäure (ALA, Vorläufer der Omega-3- und -6-Fettsäuren), Gamma-Linolensäure (GLA), Eicosadiensäure, Dihomo-Linolensäure, Eicosapentaensäure (EPA), fettlöslichen Vitaminen wie α-Tocopherol (Vitamin E), oder fettlöslichen Pigmenten wie Chlorophyll, oder Carotinoiden wie β-Carotin (Vitamin-A-Vorläufer), Lutein, Astaxanthin, Zeaxanthin, Cryptoxanthin (Vitamin-A-Vorläufer), Lycopin, Sterolen, Alkaloiden, phenolischen Verbindungen wie Flavonoiden, oder Terpenen wie Phytol (Vitamin-E-Vorläufer).

6. Verfahren nach einem der vorstehenden Ansprüche,
**dadurch gekennzeichnet, dass** die eukaryotischen Mikroalgen ausgewählt werden aus Chlorella, Nannocloropsis, Dunaliella und Euglena und **dass** die prokaryotischen Mikroalgen ausgewählt werden aus Spirulina Arthrospira platensis oder Spirulina maxima und Aphanizomenon floes-aquae.

7. Verfahren nach einem der vorstehenden Ansprüche,
**dadurch gekennzeichnet, dass** es einen zusätzlichen Fest-Flüssig-Abscheidungsschritt 5), vorzugsweise durch Zentrifugieren, des gewonnen Extrakts nach Schritt 2), 3) oder 4) umfasst.

8. Verfahren nach dem vorstehenden Anspruch,
**dadurch gekennzeichnet, dass** es einen zusätzlichen Filtrationsschritt 6) des flüssigen Teils, der durch Abscheidung des Extrakts gewonnen wird, das nach Schritt 5) gewonnen wird, umfasst.

9. Verfahren nach einem der vorstehenden Ansprüche,
**dadurch gekennzeichnet, dass** das Verhältnis Mikroalgen oder Cyanobakterien/Öl zwischen 1/5 und 1/50 des Volumens, vorzugsweise 1/20 des Volumens liegt.

10. Verwendung des Verfahrens nach einem der vorstehenden Ansprüche, wobei in dem Fall, in dem das Verfahren einen Schritt 3) der Ultraschallbehandlung umfasst, die Ultraschallleistung und die Temperatur moduliert werden, um die Art und die Menge der Verbindungen, die aus der Biomasse extrahiert werden, zu variieren.

11. Verwendung des Verfahrens nach einem der Ansprüche 1 bis 9, wobei in dem Fall, in dem das Verfahren einen Schritt 4) der Behandlung mit elektromagnetischen Mikrowellen umfasst, die Leistung, die Anzahl der Expositionen und die Temperatur moduliert werden, um die Art und die Menge der Verbindungen, die aus der Biomasse extrahiert werden, zu variieren.

12. Verwendung des Verfahrens nach einem der Ansprüche 1 bis 9, wobei die Form der ursprünglichen Biomasse, die Art und die Menge des Öls und die Art und die Menge des mindestens einen amphiphilen Zusatzstoffs und der Schritt 3) der Ultraschallbehandlung und/oder 4) der Behandlung mit elektromagnetischen Mikrowellen moduliert werden, um die fettlöslichen und wasserlöslichen Verbindungen, die aus den eukaryotischen Mikroalgen oder den Cyanobakterien extrahiert werden, zu variieren.

## Claims

1. Process for obtaining liposoluble and hydrosoluble compounds from a biomass of eukaryotic or prokaryotic microalgae, **characterized in that** said process comprises:
1. a step of mixing said biomass with oil, preferably vegetable oil, said oil comprising between 0.25% and 10% by mass of at least one amphiphilic adjuvant selected from a monoglyceride, a diglyceride, and a phospholipid, allowing the polarity of said oil to be modulated, and
3. a step of extracting said liposoluble and hydrosoluble compounds by means of an ultrasound treatment with the application of an ultrasonic power (Pus) of between 1 and 1000 W/L applied to said biomass mixed with said oil obtained in step 1) at a temperature (T) of between 15 and 70°C for a time of between 30 seconds and 1 hour, and/or
4. a step of extracting said liposoluble and hydrosoluble compounds by means of electromagnetic microwave treatment with the application of a power (W) of between 1 and 1000 W/L applied to said biomass mixed with said oil obtained in step 1) at a temperature (T) of between 15 and 70°C for a time of between 30 seconds and 1 hour.

2. Process according to the preceding claim, **characterized in that** the biomass of eukaryotic or prokaryotic microalgae is in dry, moist or pre-extracted form.

3. Process according to either one of the preceding claims, where said at least one amphiphilic adjuvant is selected from monostearin, sorbitan monooleate, sorbitan monolaurate, polyoxyethylene sorbitan monopalmitate, polyoxyethylene sorbitan monooleate, glyceryl monooleate, glyceryl palmitostearate, and soy lecithin.

4. Process according to the preceding claim, where said at least one amphiphilic adjuvant is selected from sorbitan monooleate and polyoxyethylene sorbitan monooleate.

5. Process according to any one of the preceding claims,
**characterized in that** the liposoluble compounds are selected from short-chain saturated fatty acids such as butyric acid, caproic acid or caprylic acid, long-chain saturated fatty acids such as capric acid, undecanoic acid, lauric acid, tridecanoic acid, myristyic acid, pentadecanoic acid, palmitic acid, margaric acid or stearic acid, very-long-chain saturated fatty acids such as arachidic acid, docosanoic acid, tricosanoic acid or tetracosanoic acid, monounsaturated fatty acids such as undecenoic acid, lauroleic acid, pentadecenoic acid, palmitoleic acid, heptadecenoic acid, oleic acid, eicosic acid, docosaenoic acid or tetracosenoic acid, polyunsaturated fatty acids such as hexadecadienoic acid, linoleic acid (precursor of omega-3 and omega-6), alpha-linolenic acid (ALA, precursor of omega-3 and omega-6), gamma-linolenic acid (GLA), eicosadienoic acid, dihomolinolenic acid or eicosapentaenoic acid (EPA), liposoluble vitamins such as α-tocopherol (vitamin E), liposoluble pigments such as chlorophyll, carotenoids such as β-carotene (vitamin A precursor), lutein, asthaxanthin, zeaxanthin, cryptoxanthin (vitamin A precursor) or lycopene, sterols, alkaloids, phenolic compounds such as flavonoids, and terpenes such as phytol (vitamin E precursor).

6. Process according to any one of the preceding claims,
**characterized in that** the eukaryotic microalgae are selected from Chlorella, Nannochloropsis, Dunaliella and Euglena, and **in that** the prokaryotic microalgae are selected from Arthrospira platensis spirulina, Spirulina maxima spirulina, and Aphanizomenon flos-aquae.

7. Process according to any one of the preceding claims,
**characterized in that** it comprises an additional step, step 5), of solid-liquid separation, preferably by means of centrifugation, of the extract obtained after step 2), 3) or 4).

8. Process according to the preceding claim, **characterized in that** it comprises an additional step, step 6), of filtering the liquid part obtained by separating the extract obtained after step 5).

9. Process according to any one of the preceding claims,
**characterized in that** the ratio of microalgae or cyanobacteria to oil is between 1/5^{th} and 1/50^{th} of the volume, preferably 1/20^{th} of the volume.

10. Use of the process according to any one of the preceding claims, where if the process comprises an ultrasound treatment step, step 3), the ultrasonic power and the temperature are modulated so as to vary the nature and the quantity of the compounds extracted from said biomass.

11. Use of the process according to any one of claims 1 to 9, where if the process comprises an electromagnetic microwave treatment step, step 4), the power, the number of exposures and the temperature are modulated so as to vary the nature and quantity of the compounds extracted from said biomass.

12. Use of the process according to any one of claims 1 to 9, where the form of the original biomass, the nature and quantity of the oil and the nature and quantity of said at least one amphiphilic adjuvant, and step 3) of ultrasound treatment and/or step 4) of electromagnetic microwave treatment are modulated so as to vary the liposoluble and hydrosoluble compounds extracted from said eukaryotic microalgae or cyanobacteria.
